# EUROPEAN PATENT APPLICATION

(11) **EP 4 344 647 A1**
(43) Date of publication of application: **03.04.2024**
(21) Application number: 23198383.4
(22) Date of filing: 19.09.2023
(51) Int. Cl.: A61B 8/00, A61B 8/08

(54) **ULTRASOUND DIAGNOSTIC SYSTEM AND CONTROL METHOD FOR ULTRASOUND DIAGNOSTIC SYSTEM**

(30) Priority: 28.09.2022 JP 2022154898
(71) Applicant: FUJIFILM Corporation, Tokyo 106-8620 (JP)
(72) Inventor: TASHIRO, Rika, Kanagawa, 258-8538 (JP)
(74) Representative: Hughes, Andrea Michelle

(57) **Abstract**

Provided are an ultrasound diagnostic system and a control method for an ultrasound diagnostic system that enable a user to smoothly examine a subject.

An ultrasound diagnostic system includes: a subject specification unit (30) that specifies a subject using identification information of the subject; an observation site selection unit (25) that selects an observation site; a monitor (23) that has a first display region and a second display region; a display controller (22) that displays a captured ultrasound image in the first display region; a reference image display unit (26) that displays a reference image for the selected observation site in the second display region; an image memory (24) that stores a past ultrasound image of the subject; and a past image display unit (29) that, in a case where the past ultrasound image of the observation site of the specified subject is stored in the image memory (24), reads out the past ultrasound image from the image memory (24) and displays the past ultrasound image in the second display region instead of the reference image.

## Description

### BACKGROUND OF THE INVENTION

### 1. Field of the Invention

The present invention relates to an ultrasound diagnostic system and a control method for an ultrasound diagnostic system for displaying an ultrasound image of an observation site of a subject.

### 2. Description of the Related Art

Conventionally, an observation site has been examined by capturing an ultrasound image representing a tomogram in a subject using a so-called ultrasound diagnostic system. A user of the ultrasound diagnostic system normally moves an ultrasound probe to a position where an ultrasound image of a target site can be captured while confirming the captured ultrasound image. However, depending on the user's level of proficiency, for example, the site of the subject that is currently being imaged may not be accurately grasped even in a case of confirming the ultrasound image, and the target observation site may not be easily imaged. This may make it difficult to smoothly perform the examination.

In that respect, for example, a technology disclosed in JP2015-131099A has been developed such that a user can smoothly perform an examination. JP2015-131099A discloses an ultrasound diagnostic system that displays, side by side, an ultrasound image captured in a past and an ultrasound image currently being captured for the same subject. The user can move an ultrasound probe with reference to the past ultrasound image.

### SUMMARY OF THE INVENTION

However, in the technology of JP2015-131099A, in a case of examining the subject for which the past ultrasound image is not present because the examination is not performed in the past or the like, the user performs the examination while confirming the current ultrasound image. Therefore, depending on the level of proficiency, for example, the target observation site may not be easily imaged. This may make it difficult to smoothly perform the examination.

The present invention has been made in order to solve such a conventional problem, and an object of the present invention is to provide an ultrasound diagnostic system and a control method for an ultrasound diagnostic system that enable a user to smoothly examine a subj ect.

According to the following configuration, the above-described object can be achieved.
[1] An ultrasound diagnostic system that displays an ultrasound image of an observation site of a subject captured by scanning with an ultrasound probe, the ultrasound diagnostic system comprising:
   a subject specification unit configured to specify the subject in response to an input of identification information of the subject;
   an observation site selection unit configured to select the observation site;
   a monitor having a first display region and a second display region;
   a display controller configured to display the captured ultrasound image in the first display region;
   a reference image display unit configured to display, in the second display region, a reference image for the observation site selected by the observation site selection unit;
   an image memory configured to store a past ultrasound image of the subject; and
   a past image display unit configured to, in a case where it is determined whether or not the past ultrasound image of the observation site of the subject specified by the subject specification unit is stored in the image memory, and the past ultrasound image is stored, read out the past ultrasound image from the image memory and display the past ultrasound image in the second display region instead of the reference image.
[2] The ultrasound diagnostic system according to [1],
   in which the past image display unit is configured to, in a case where a plurality of the past ultrasound images are stored in the image memory, select a past ultrasound image closest to the ultrasound image being displayed in the first display region from the plurality of past ultrasound images and display the selected past ultrasound image in the second display region.
[3] The ultrasound diagnostic system according to [1] or [2],
   in which the monitor has a third display region different from the first display region and the second display region, and
   the ultrasound diagnostic system further comprises a scanning guide display unit configured to display, in the third display region, a scanning guide for guiding scanning of the ultrasound probe for the observation site selected by the observation site selection unit.
[4] The ultrasound diagnostic system according to [3], in which the scanning guide is a video.
[5] The ultrasound diagnostic system according to [3],
   in which the scanning guide is an optical image in which the subject and the ultrasound probe during the scanning of the observation site of the subject are captured.
[6] The ultrasound diagnostic system according to [5], further comprising:
   an optical camera configured to acquire the optical image.
[7] The ultrasound diagnostic system according to any one of [1] to [6],
   in which the observation site is any one of an entire large intestine, a colon, or a rectum.
[8] The ultrasound diagnostic system according to [7], further comprising:
   a feces detection unit configured to detect a feces from the ultrasound image, and
   in which the display controller is configured to highlight and display, in the ultrasound image, a position of the feces detected by the feces detection unit.
[9] The ultrasound diagnostic system according to [8],
   in which the feces detection unit is configured to detect the feces by using a trained model that has been trained using ultrasound images of large intestines of a large number of subjects.
[10] A control method for an ultrasound diagnostic system, the control method being for displaying an ultrasound image of an observation site of a subject captured by scanning with an ultrasound probe, comprising:
   specifying the subject in response to an input of identification information of the subject;
   selecting the observation site;
   displaying the captured ultrasound image in a first display region of a monitor;
   displaying a reference image for the selected observation site in a second display region of the monitor;
   storing a past ultrasound image of the subject in an image memory; and
   in a case where it is determined whether or not the past ultrasound image of the observation site of the specified subject is stored in the image memory, and the past ultrasound image is stored, reading out the past ultrasound image from the image memory and displaying the past ultrasound image in the second display region instead of the reference image.

According to the present invention, an ultrasound diagnostic system comprises: a subject specification unit that specifies a subject in response to an input of identification information of the subject; an observation site selection unit that selects an observation site; a monitor that has a first display region and a second display region; a display controller that displays a captured ultrasound image in the first display region; a reference image display unit that displays, in the second display region, a reference image for the observation site selected by the observation site selection unit; an image memory that stores a past ultrasound image of the subject; and a past image display unit that, in a case where it is determined whether or not the past ultrasound image of the observation site of the subject specified by the subject specification unit is stored in the image memory, and the past ultrasound image is stored, reads out the past ultrasound image from the image memory and displays the past ultrasound image in the second display region instead of the reference image. Therefore, the user can smoothly examine the subject.

### BRIEF DESCRIPTION OF THE DRAWINGS

Fig. 1 is a block diagram showing a configuration of an ultrasound diagnostic system according to Embodiment 1 of the present invention.
Fig. 2 is a block diagram showing a configuration of a transmission and reception circuit in Embodiment 1 of the present invention.
Fig. 3 is a block diagram showing a configuration of an image generation unit in Embodiment 1 of the present invention.
Fig. 4 is a diagram showing an example of a menu screen for selecting an observation site.
Fig. 5 is a diagram showing examples of a first display region, a second display region, and a third display region.
Fig. 6 is a diagram showing an example in which a current ultrasound image is displayed in the first display region, a reference image is displayed in the second display region, and a video of a scanning guide is displayed in the third display region.
Fig. 7 is a diagram showing an example of an icon for selecting an annotation to be added to an ultrasound image.
Fig. 8 is an example showing an example of a past ultrasound image displayed in the second display region.
Fig. 9 is a diagram showing an example in which the current ultrasound image is displayed in the first display region, the past ultrasound image is displayed in the second display region, and the video of the scanning guide is displayed in the third display region.
Fig. 10 is a flowchart showing an operation of the ultrasound diagnostic system according to Embodiment 1 of the present invention.
Fig. 11 is a block diagram showing a configuration of an ultrasound diagnostic system according to Embodiment 2 of the present invention.
Fig. 12 is a diagram showing an example of an optical image displayed in the third display region as the scanning guide.
Fig. 13 is a diagram showing an example in which the current ultrasound image is displayed in the first display region, the past ultrasound image is displayed in the second display region, and the optical image is displayed in the third display region.

### DESCRIPTION OF THE PREFERRED EMBODIMENTS

Hereinafter, embodiments of the present invention will be described with reference to the accompanying drawings.

The description of configuration requirements to be described below is made based on a representative embodiment of the present invention, but the present invention is not limited to such an embodiment.

In the present specification, a numerical range represented by "to" means a range including numerical values described before and after "to" as a lower limit value and an upper limit value, respectively.

In the present specification, "same" and "identical" include an error range generally allowed in the technical field.

### Embodiment 1

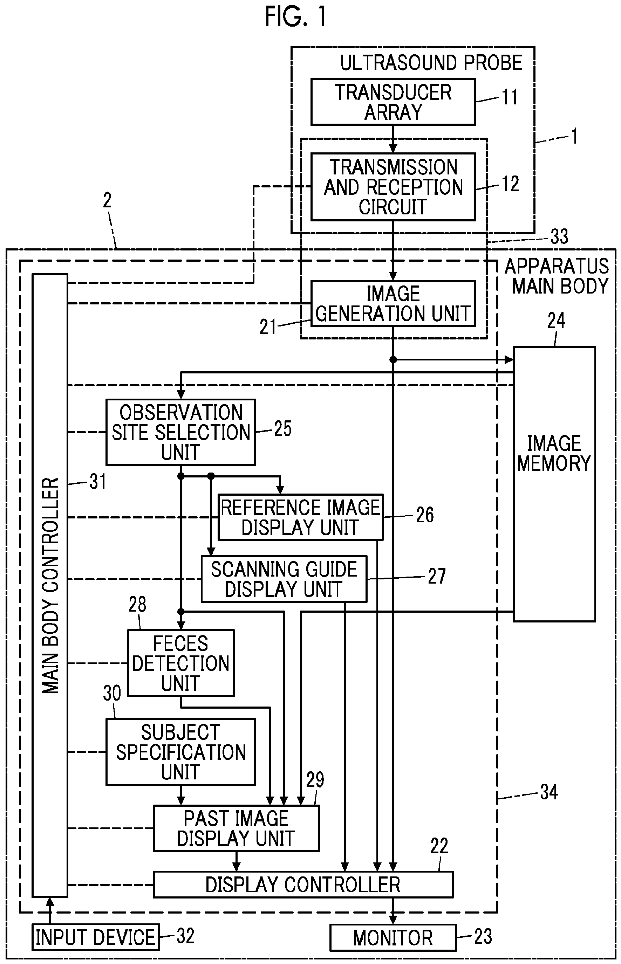

Fig. 1 shows a configuration of an ultrasound diagnostic system according to Embodiment 1 of the present invention. The ultrasound diagnostic system comprises an ultrasound probe 1 and an apparatus main body 2 connected to the ultrasound probe 1.

The ultrasound probe 1 includes a transducer array 11. A transmission and reception circuit 12 is connected to the transducer array 11.

The apparatus main body 2 includes an image generation unit 21 connected to the transmission and reception circuit 12 of the ultrasound probe 1. A display controller 22 and a monitor 23 are sequentially connected to the image generation unit 21. In addition, an image memory 24 is connected to the image generation unit 21. An observation site selection unit 25 is connected to the image memory 24. A reference image display unit 26, a scanning guide display unit 27, and a feces detection unit 28 are connected to the observation site selection unit 25. The reference image display unit 26 and the scanning guide display unit 27 are connected to the display controller 22. Further, the apparatus main body 2 comprises a subject specification unit 30. A past image display unit 29 is connected to the image memory 24, the observation site selection unit 25, the feces detection unit 28, and the subject specification unit 30. The past image display unit 29 is connected to the display controller 22. Further, a main body controller 31 is connected to the transmission and reception circuit 12, the image generation unit 21, the display controller 22, the image memory 24, the observation site selection unit 25, the reference image display unit 26, the scanning guide display unit 27, the feces detection unit 28, the past image display unit 29, and the subject specification unit 30. An input device 32 is connected to the main body controller 31.

Further, the transmission and reception circuit 12 and the image generation unit 21 constitute an image acquisition unit 33. Further, the image generation unit 21, the display controller 22, the observation site selection unit 25, the reference image display unit 26, the scanning guide display unit 27, the feces detection unit 28, the past image display unit 29, the subject specification unit 30, and the main body controller 31 constitute a processor 34 for the apparatus main body 2.

The transducer array 11 of the ultrasound probe 1 includes a plurality of ultrasound transducers one-dimensionally or two-dimensionally arranged. Each of these ultrasound transducers transmits an ultrasound wave in accordance with a drive signal supplied from the transmission and reception circuit 12 and receives an ultrasound echo from the subject to output a signal based on the ultrasound echo. For example, each ultrasound transducer is composed of a piezoelectric body consisting of a piezoelectric ceramic represented by lead zirconate titanate (PZT), a polymer piezoelectric element represented by poly vinylidene di fluoride (PVDF), a piezoelectric single crystal represented by lead magnesium niobate-lead titanate (PMN-PT), or the like, and electrodes formed at both ends of the piezoelectric body.

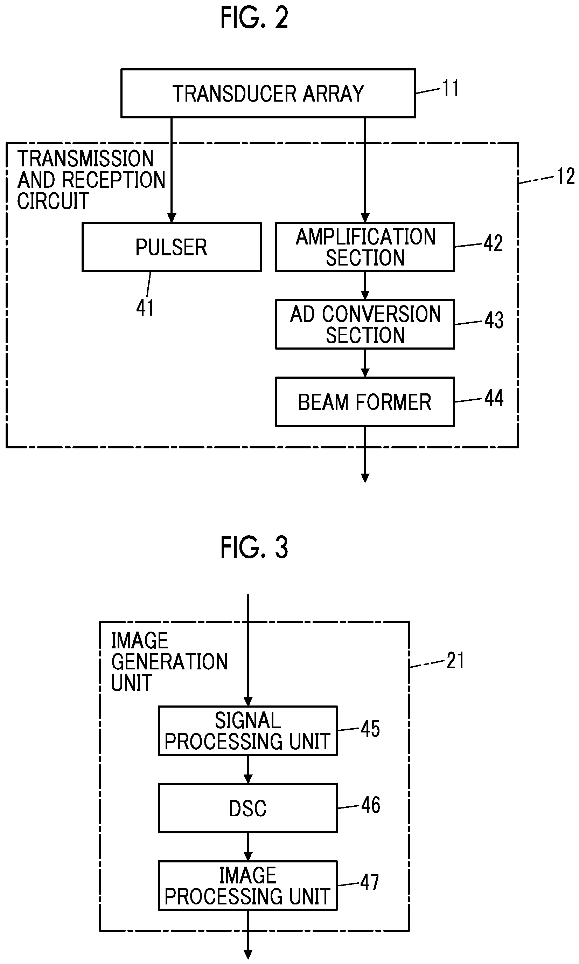

The transmission and reception circuit 12 transmits the ultrasound wave from the transducer array 11 and generates a sound ray signal based on a reception signal acquired by the transducer array 11, under the control of the main body controller 31. As shown in Fig. 2, the transmission and reception circuit 12 includes a pulsar 41 connected to the transducer array 11, and an amplification section 42, an analog-to-digital (AD) conversion section 43, and a beam former 44 that are sequentially connected in series to the transducer array 11.

The pulsar 41 includes, for example, a plurality of pulse generators, and adjusts an amount of delay of each of drive signals and supplies the drive signals to the plurality of ultrasound transducers such that ultrasound waves transmitted from the plurality of ultrasound transducers of the transducer array 11 form an ultrasound beam based on a transmission delay pattern selected according to a control signal from the main body controller 31. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, whereby an ultrasound beam is formed from the combined wave of these ultrasound waves.

The transmitted ultrasound beam is reflected in, for example, a target such as a site of the subject and propagates toward the transducer array 11 of the ultrasound probe 1. The ultrasound echo, which propagates toward the transducer array 11 in this way, is received by each of the ultrasound transducers constituting the transducer array 11. In this case, each of the ultrasound transducers constituting the transducer array 11 receives the propagating ultrasound echo to expand and contract to generate a reception signal, which is an electrical signal, and outputs these reception signals to the amplification section 42.

The amplification section 42 amplifies the signal input from each of the ultrasound transducers constituting the transducer array 11 and transmits the amplified signal to the AD conversion section 43. The AD conversion section 43 converts the signal transmitted from the amplification section 42 into digital reception data. The beam former 44 performs so-called reception focus processing by applying and adding a delay to each reception data received from the AD conversion section 43. By this reception focus processing, each reception data converted by the AD conversion section 43 is phase-added, and a sound ray signal in which the focus of the ultrasound echo is narrowed down is acquired.

As shown in Fig. 3, the image generation unit 21 has a configuration in which a signal processing section 45, a digital scan converter (DSC) 46, and an image processing section 47 are sequentially connected in series.

The signal processing section 45 generates a B-mode image signal, which is tomographic image information regarding tissues inside the subject, by performing, on the sound ray signal received from the transmission and reception circuit 12, correction of the attenuation due to the distance according to the depth of the reflection position of the ultrasound wave using a sound velocity value set by the main body controller 31 and then performing envelope detection processing.

The DSC 46 converts (raster-converts) the B-mode image signal generated by the signal processing section 45 into an image signal in accordance with a normal television signal scanning method.

The image processing section 47 performs various types of necessary image processing such as gradation processing on the B-mode image signal input from the DSC 46 and then transmits the B-mode image signal to the display controller 22 and the image memory 24. Hereinafter, the B-mode image signal that has been subjected to image processing by the image processing section 47 is referred to as an ultrasound image.

The display controller 22 performs predetermined processing on the ultrasound image or the like generated by the image generation unit 21 and displays the ultrasound image or the like on the monitor 23, under the control of the main body controller 31.

The monitor 23 performs various kinds of display under the control of the display controller 22. The monitor 23 can include a display device, such as a liquid crystal display (LCD) or an organic electroluminescence (EL) display, for example.

The main body controller 31 controls each unit of the apparatus main body 2 and the ultrasound probe 1 in accordance with a program recorded in advance, or the like.

The input device 32 accepts an input operation from an examiner and sends out input information to the main body controller 31. The input device 32 is composed of a device for the examiner to perform an input operation, such as a keyboard, a mouse, a trackball, a touchpad, or a touch panel, for example.

The image memory 24 stores the ultrasound image generated by the image generation unit 21 in association with identification information of the subject input by the user of the ultrasound diagnostic system via, for example, the input device 32 under the control of the main body controller 31. Here, as the identification information of the subject, for example, an identifier (ID) set for each subject, a subject name, or the like can be used. The image memory 24 can accumulate and store ultrasound images generated in the past examination in addition to the ultrasound image generated in the current examination.

Here, as the image memory 24, for example, recording media such as a flash memory, a hard disk drive (HDD), a solid state drive (SSD), a flexible disk (FD), a magneto-optical disk (MO disk), a magnetic tape (MT), a random access memory (RAM), a compact disc (CD), a digital versatile disc (DVD), a secure digital card (SD card), or a universal serial bus memory (USB memory) can be used.

The subject specification unit 30 specifies the subject based on the identification information of the subject input by the user via, for example, the input device 32.

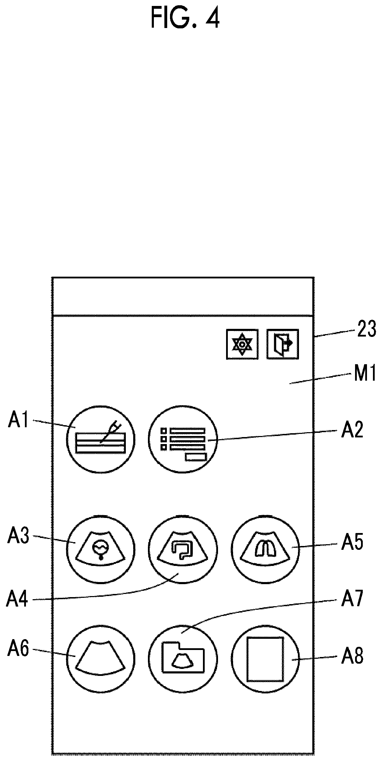

The observation site selection unit 25 selects one observation site from among a plurality of observation sites set in advance, based on an instruction input by the user via the input device 32. For example, in a menu screen M1, as shown in Fig. 4, displayed on the monitor 23 by the display controller 22, in a case where an icon related to the observation site is selected by the user via the input device 32 from among a plurality of icons A1 to A8 included in the menu screen M1, the observation site selection unit 25 can select the observation site based on the icon selected by the user.

The menu screen M1 of Fig. 4 includes an icon A1 for activating a mode in which needle puncture into a blood vessel is performed, an icon A2 for activating a mode in which a list of an examination menu is set, an icon A3 for activating a mode in which a bladder of the subject is observed and a urine volume within the bladder is measured, an icon A4 for activating a mode in which a large intestine is observed, an icon A5 for activating a mode in which a lung echo is observed, an icon A6 for starting scanning of the ultrasound probe 1, an icon A7 for displaying an examination history on the monitor 23, and an icon A8 for displaying a user manual of the ultrasound diagnostic system on the monitor 23.

For example, among these icons A1 to A8, the observation site selection unit 25 can select a blood vessel as the observation site in a case where the icon A1 is selected, select the bladder as the observation site in a case where the icon A3 is selected, select the large intestine as the observation site in a case where the icon A4 is selected, and select the lung as the observation site in a case where the icon A5 is selected. The observation site selection unit 25 can select any one of the entire large intestine, the colon, or the rectum as the observation site, particularly in a case where the icon A4 is selected. Here, it is possible for the user to set in advance whether the observation site selection unit 25 selects the entire large intestine, the colon, or the rectum by selecting the icon A4.

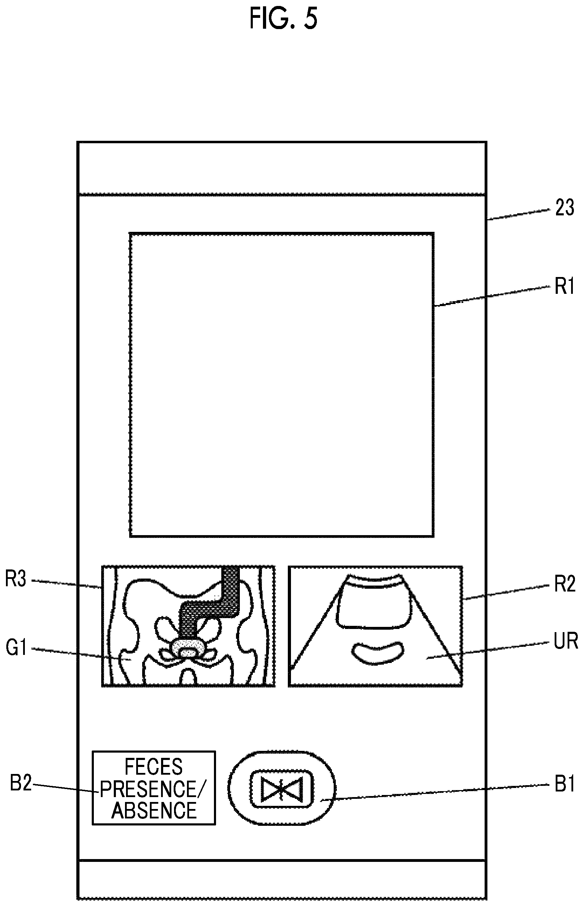

In a case where the observation site is selected by the observation site selection unit 25, the main body controller 31 transitions the display of the monitor 23 to, for example, the display as shown in Fig. 5. In this case, the monitor 23 has a first display region R1, a second display region R2, and a third display region R3.

The display controller 22 sequentially displays latest ultrasound images generated by the image generation unit 21 in the first display region R1 of the monitor 23.

The reference image display unit 26 stores a reference image for each of a plurality of predetermined observation sites and displays a reference image UR for the observation site selected by the observation site selection unit 25 in the second display region R2 of the monitor 23, as shown in Fig. 5, for example. Here, the reference image UR refers to a standard ultrasound image in which the observation site is captured. The user can easily image the target observation site by confirming the current ultrasound image displayed in the first display region R1 and the reference image UR displayed in the second display region R2 and by performing scanning while moving the ultrasound probe 1 such that an ultrasound image similar to the reference image UR is obtained.

The scanning guide display unit 27 stores, for each of the plurality of predetermined observation sites, a scanning guide for guiding the scanning of the ultrasound probe 1 in order to image the observation site, and displays a scanning guide G1 for the observation site selected by the observation site selection unit 25 in the third display region R3 of the monitor 23 as shown in Fig. 5, for example. Here, the scanning guide display unit 27 can display, for example, a video or a still image showing a movement path of the ultrasound probe 1 to the observation site, as the scanning guide G1. The user can easily grasp the movement path of the ultrasound probe 1 by confirming the scanning guide G1 displayed in the third display region R3 and can easily image the target observation site.

Here, as shown in Fig. 5, the monitor 23 can include a freeze button B1 and a feces presence/absence button B2 for discriminating the presence or absence of a feces, in the display having the first display region R1, the second display region R2, and the third display region R3. The freeze button B1 is a button for temporarily pausing the continuous display of the ultrasound image in the first display region R1, that is, for so-called freezing. The feces presence/absence button B2 is a button for executing processing of detecting a feces present in the large intestine of the subject through the feces detection unit 28, as will be described below.

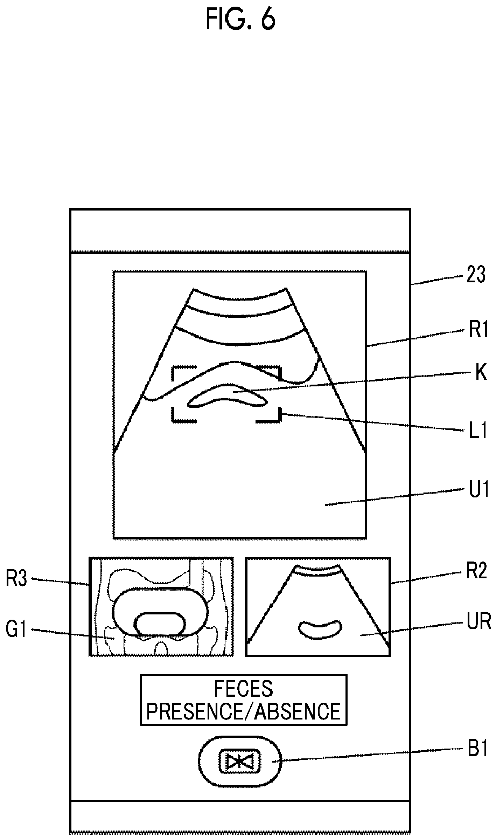

The feces detection unit 28 detects a feces K from an ultrasound image U1 by analyzing the ultrasound image generated by the image generation unit 21, as shown in Fig. 6. The feces detection unit 28 can detect the feces K using, for example, a so-called template matching method of storing a plurality of template images related to the feces K and searching the ultrasound image U1 using the plurality of template images. The feces detection unit 28 can also detect the feces K from the ultrasound image U1 using, for example, a trained model in so-called machine learning in which training has been performed using ultrasound images of the large intestines of a large number of subjects, which show the feces K.

The feces detection unit 28 can execute processing of detecting the feces K on the ultrasound image U1 continuously displayed in the first display region R1, for example, with the user's selection of the feces presence/absence button B2 displayed on the monitor 23 via the input device 32 as a trigger.

In a case where the feces K is detected by the feces detection unit 28, the display controller 22 can highlight and display the position of the feces K, for example, by displaying a region-of-interest suggestion line L1 shown in Fig. 6. The region-of-interest suggestion line L1 is a line suggesting the presence of a region including the detected feces K and can have any form, but for example, it can consist of four bent lines representing four corners of a rectangle and surrounding the feces K. In addition to displaying the region-of-interest suggestion line L1, the display controller 22 can also highlight and display the position of the feces K using, for example, a display aspect different from that of the surroundings, such as displaying a color of an image of the feces K in a color different from the surroundings, displaying a contour line of the feces K, and making the feces K blink.

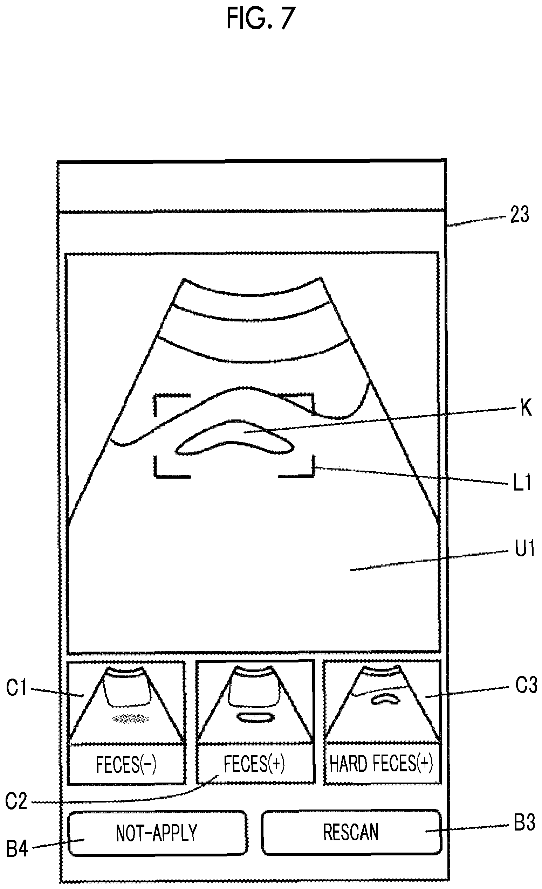

In a case where the freeze button B1 is selected by the user via the input device 32 in a state in which the feces K is detected by the feces detection unit 28, the main body controller 31 can transition the display of the monitor 23 to, for example, the display as shown in Fig. 7. In this display, for example, the latest ultrasound image U1 in which the feces K is detected is displayed on the monitor 23, and annotation icons C1, C2, and C3 for adding annotations related to properties of the feces K to the ultrasound image U1 are displayed. The user can add the annotation corresponding to the selected annotation icon to the ultrasound image U1 by selecting any one of the annotation icon C1, C2, or C3 via the input device 32. The annotation added to the ultrasound image U1 in this manner is stored in the image memory 24 in association with the ultrasound image U1. Further, in the display of Fig. 7, the monitor 23 can also include a not-apply button B3 for issuing an instruction not to add the annotation to the ultrasound image U1 from the fact that the property of the detected feces K is not applied to the properties of the feces K corresponding to the annotation icons C1, C2, and C3, and a rescan button B4 for restarting the scanning by releasing the freeze.

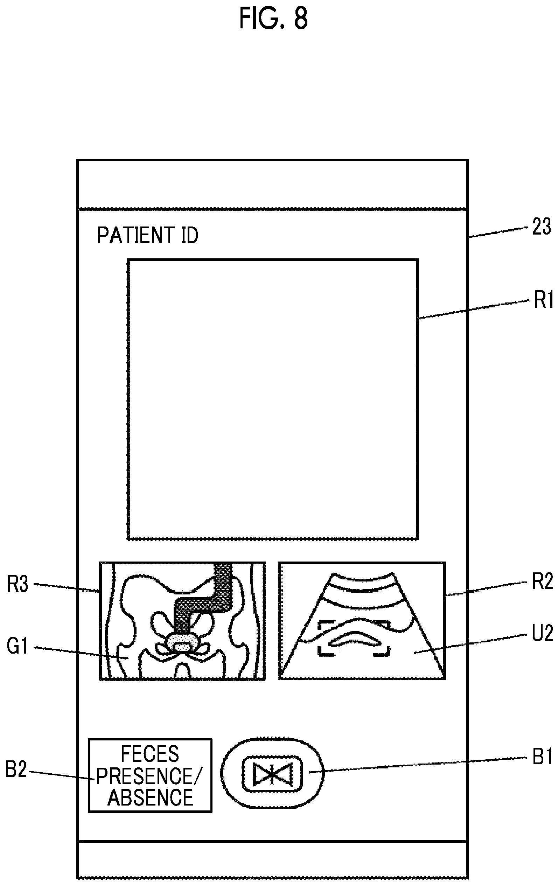

The past image display unit 29 determines whether or not the past ultrasound image of the observation site of the subject specified by the subject specification unit 30 is stored in the image memory 24. The past image display unit 29 reads out the past ultrasound image from the image memory 24 in a case where the past ultrasound image of the subject is stored in the image memory 24, and displays a past ultrasound image U2 in the second display region R2 instead of the reference image UR as shown in Fig. 8.

Since the shape, size, position, and the like of the observation site differ for each subject, using the past ultrasound image U2 of the same subject as a reference of the scanning allows for easier imaging of the observation site rather than using the reference image UR, which is the standard ultrasound image, as a reference of the scanning.

Here, in a case where a plurality of past ultrasound images U2 are stored in the image memory 24, the past image display unit 29 can select an ultrasound image U2 that is closest to, that is, most similar to, the ultrasound image U1 being displayed in the first display region R1 from the plurality of past ultrasound images U2, and can display the selected ultrasound image U2 in the second display region R2. In this case, the past image display unit 29 can calculate a plurality of similarities between the ultrasound image U1 being displayed in the first display region R1 and the plurality of past ultrasound images U2, and can select a past ultrasound image U2 having the greatest similarity to the ultrasound image U1 as the ultrasound image U2 closest to the ultrasound image U1. The user can easily grasp whether or not the site of the subject, which is currently being imaged, is the target observation site by comparing the past ultrasound image U2 and the current ultrasound image U1 displayed in this manner.

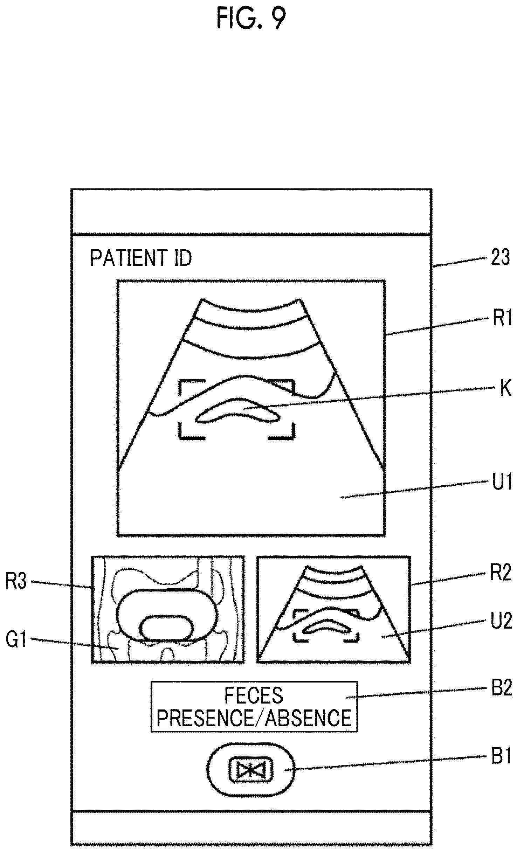

Further, as shown in Fig. 9, in a case where the detection processing of the feces K by the feces detection unit 28 is executed on the current ultrasound image U1 and the feces K is detected, the past image display unit 29 can select an ultrasound image U2 in which the feces K is detected, from among the plurality of past ultrasound images U2, and can display the ultrasound image U2 in the second display region R2.

In addition, in a case where the feces K is detected in the read-out past ultrasound image U2, the past image display unit 29 can also enlarge and display the ultrasound image U2 centered on the position of the detected feces K in the second display region R2. As a result, the user can clearly confirm the region including the feces K.

Although the processor 34 including the image generation unit 21, the display controller 22, the observation site selection unit 25, the reference image display unit 26, the scanning guide display unit 27, the feces detection unit 28, the past image display unit 29, the subject specification unit 30, and the main body controller 31 may be composed of a central processing unit (CPU) and a control program for causing the CPU to perform various types of processing, the processor 34 may be composed of a field programmable gate array (FPGA), a digital signal processor (DSP), an application specific integrated circuit (ASIC), a graphics processing unit (GPU), or other integrated circuits (ICs), or may be composed of a combination thereof.

In addition, the image generation unit 21, the display controller 22, the observation site selection unit 25, the reference image display unit 26, the scanning guide display unit 27, the feces detection unit 28, the past image display unit 29, the subject specification unit 30, and the main body controller 31 of the processor 34 can also be configured by being integrated partially or entirely into one CPU or the like.

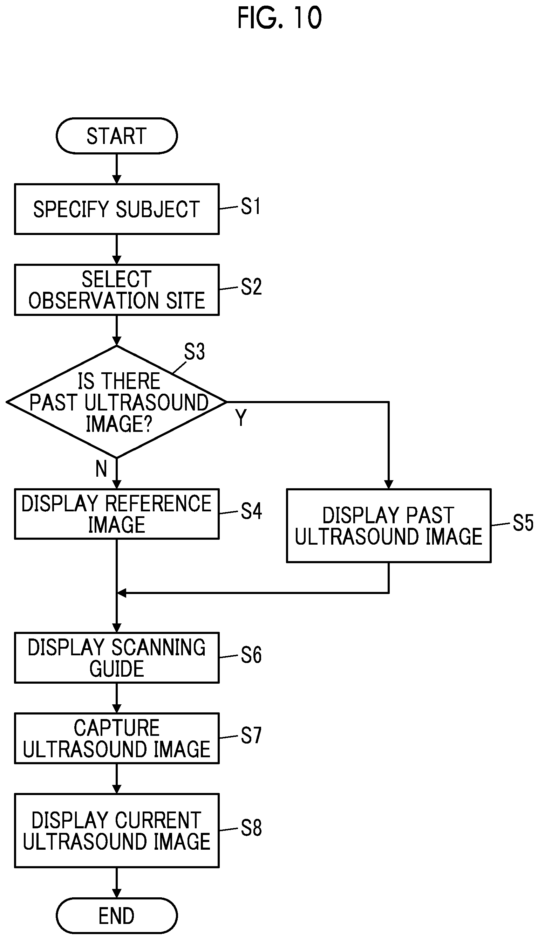

Next, an example of an operation of the ultrasound diagnostic system according to Embodiment 1 will be described using the flowchart of Fig. 10.

First, in step S1, the subject specification unit 30 specifies the subject to be examined based on the identification information, such as an ID or a name of the subject, input by the user via the input device 32.

Next, in step S2, the observation site selection unit 25 selects one observation site from among the plurality of observation sites set in advance, based on an instruction input by the user via the input device 32. For example, in a menu screen M1, as shown in Fig. 4, displayed on the monitor 23 by the display controller 22, in a case where an icon related to the observation site is selected by the user via the input device 32 from among a plurality of icons A1 to A8 included in the menu screen M1, the observation site selection unit 25 can select the observation site based on the icon selected by the user.

In a case where the observation site is selected in step S2, the main body controller 31 transitions the display of the monitor 23 to the display including the first display region R1, the second display region R2, and the third display region R3 as shown in Fig. 5.

In step S3, the past image display unit 29 refers to the image memory 24 to determine whether or not the past ultrasound image U2 of the subject specified in step S1 is stored in the image memory 24.

In a case where it is determined that the past ultrasound image U2 of the subject is not stored in the image memory 24, the process proceeds to step S4. In step S4, the reference image display unit 26 displays the reference image UR for the observation site selected by the observation site selection unit 25 in the second display region R2 of the monitor 23, as shown in Fig. 5.

In step S6 following step S4, the scanning guide display unit 27 displays the scanning guide G1 for the observation site selected by the observation site selection unit 25 in the third display region R3 of the monitor 23, as shown in Fig. 5.

In step S7, the user performs the scanning while moving the ultrasound probe 1 on a body surface of the subject, whereby a plurality of frames of the ultrasound images U1 are continuously captured. In this case, the transducer array 11 of the ultrasound probe 1 transmits the ultrasound beam into the subject and receives the ultrasound echo from the inside of the subject, thereby generating the reception signal. The transmission and reception circuit 12 of the image acquisition unit 33 performs so-called reception focus processing on the reception signal to generate the sound ray signal, under the control of the main body controller 31. The sound ray signal generated by the transmission and reception circuit 12 is sent out to the image generation unit 21. The image generation unit 21 generates the ultrasound image using the sound ray signal sent out from the transmission and reception circuit 12.

In step S8, the display controller 22 sequentially displays the plurality of frames of the ultrasound images U1 obtained in step S7 in the first display region R1 of the monitor 23. The user can easily image the target observation site by confirming the current ultrasound image displayed in the first display region R1 and the reference image UR displayed in the second display region R2 and by performing scanning while moving the ultrasound probe 1 such that an ultrasound image similar to the reference image UR is obtained. Further, the user can easily grasp the movement path of the ultrasound probe 1 by confirming the scanning guide G1 displayed in the third display region R3 and can easily image the target observation site.

In a case where it is determined that the past ultrasound image U2 of the subject is stored in the image memory 24, the process proceeds to step S5. In step S5, the past image display unit 29 reads out the past ultrasound image from the image memory 24 and displays the past ultrasound image U2 in the second display region R2 instead of the reference image UR as shown in Fig. 8. In a case where the plurality of past ultrasound images U2 are stored in the image memory 24, the past image display unit 29 can select an ultrasound image U2 that is closest to, that is, most similar to, the ultrasound image U1 being displayed in the first display region R1 from the plurality of past ultrasound images U2, and can display the selected ultrasound image U2 in the second display region R2.

In step S6 following step S5, the scanning guide display unit 27 displays the scanning guide G1 for the observation site selected by the observation site selection unit 25 in the third display region R3 of the monitor 23, as shown in Fig. 5.

Subsequently, in step S7, the user performs the scanning while moving the ultrasound probe 1 on the body surface of the subject, whereby the plurality of frames of the ultrasound images U1 are continuously captured.

Finally, in step S8, the display controller 22 sequentially displays the plurality of frames of the ultrasound images U1 obtained in step S7 in the first display region R1 of the monitor 23. Since the shape, size, position, and the like of the observation site differ for each subject, using the past ultrasound image U2 of the same subject as a reference of the scanning allows for easier imaging of the observation site rather than using the reference image UR, which is the standard ultrasound image, as a reference of the scanning.

In a case where the processing of step S8 is completed in this manner, the operation of the ultrasound diagnostic system in accordance with the flowchart of Fig. 10 is completed.

From the above, with the ultrasound diagnostic system of Embodiment 1, the reference image UR is displayed in the second display region R2 together with the current ultrasound image U1 displayed in the first display region R1 in a case where the past ultrasound image U2 of the observation site of the subject specified by the subject specification unit 30 is not stored in the image memory 24, and the past ultrasound image U2 is displayed in the second display region R2 instead of the reference image UR together with the current ultrasound image U1 displayed in the first display region R1 in a case where the past ultrasound image U2 of the observation site of the subject specified by the subject specification unit 30 is stored in the image memory 24. Therefore, the user can easily image the target observation site by confirming the reference image UR or the past ultrasound image U2 regardless of the presence or absence of the past ultrasound image U2 of the subject, and can smoothly perform the examination.

Although a case where the transmission and reception circuit 12 is provided in the ultrasound probe 1 has been described, the transmission and reception circuit 12 may be provided in the apparatus main body 2.

In addition, although a case where the image generation unit 21 is provided in the apparatus main body 2 has been described, the image generation unit 21 may be provided in the ultrasound probe 1.

Further, the apparatus main body 2 may be a so-called stationary type, a portable type that is easy to carry, or a so-called handheld type that is composed of, for example, a smartphone or a tablet type computer. As described above, the type of the device that constitutes the apparatus main body 2 is not particularly limited.

Further, although a case where the apparatus main body 2 comprises the image memory 24 has been described, the image memory 24 may be a memory externally attached to the apparatus main body 2. In addition, the ultrasound diagnostic system can comprise, for example, a server connected to the apparatus main body 2 via a network. In this case, the image memory 24 may be provided in the server instead of the apparatus main body 2.

Further, in the flowchart of Fig. 10, although the subject is specified in step S1 and then the observation site is selected in step S2, it is also possible to specify the subject after selecting the observation site.

### Embodiment 2

In Embodiment 1, the scanning guide display unit 27 displays a video or a still image showing the movement path of the ultrasound probe 1 to the observation site as the scanning guide G1, but an optical image showing the ultrasound probe 1 disposed on the subject can also be used as the scanning guide G1.

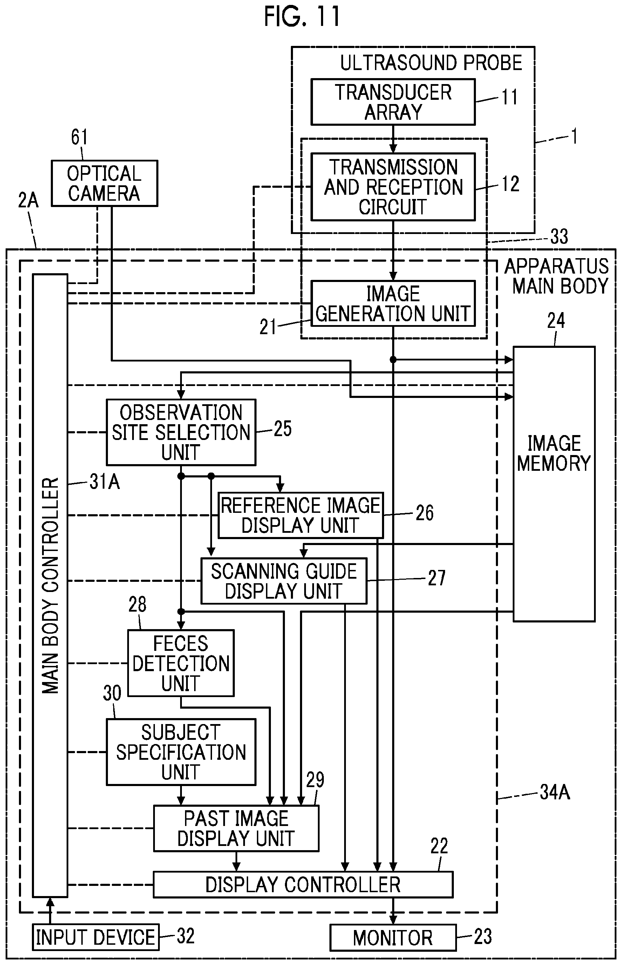

Fig. 11 shows a configuration of an ultrasound diagnostic system of Embodiment 2. The ultrasound diagnostic system of Embodiment 2 comprises an apparatus main body 2A instead of the apparatus main body 2 and further comprises an optical camera 61 with respect to the ultrasound diagnostic system of Embodiment 1 shown in Fig. 1. The apparatus main body 2A comprises a main body controller 31A instead of the main body controller 31 with respect to the apparatus main body 2 of Embodiment 1.

In the apparatus main body 2A, the image memory 24 and the main body controller 31A are connected to the optical camera 61. Further, the image memory 24 is connected to the scanning guide display unit 27. Further, the image generation unit 21, the display controller 22, the observation site selection unit 25, the reference image display unit 26, the scanning guide display unit 27, the feces detection unit 28, the past image display unit 29, the subject specification unit 30, and the main body controller 31A constitute a processor 34A for the apparatus main body 2A.

The optical camera 61 includes, for example, an image sensor, such as a so-called charge coupled device (CCD) image sensor or a so-called a complementary metal-oxide-semiconductor (CMOS) image sensor, and optically images the subject and the ultrasound probe 1 disposed on the body surface of the subject during the scanning of the observation site of the subject to acquire the optical image, under the control of the main body controller 31A. The optical camera 61 transmits the acquired optical image to the image memory 24.

The image memory 24 stores the ultrasound image U1 and the optical image acquired by the image acquisition unit 33 and the optical camera 61 at the same timing or the closest timing in association with each other, under the control of the main body controller 31A.

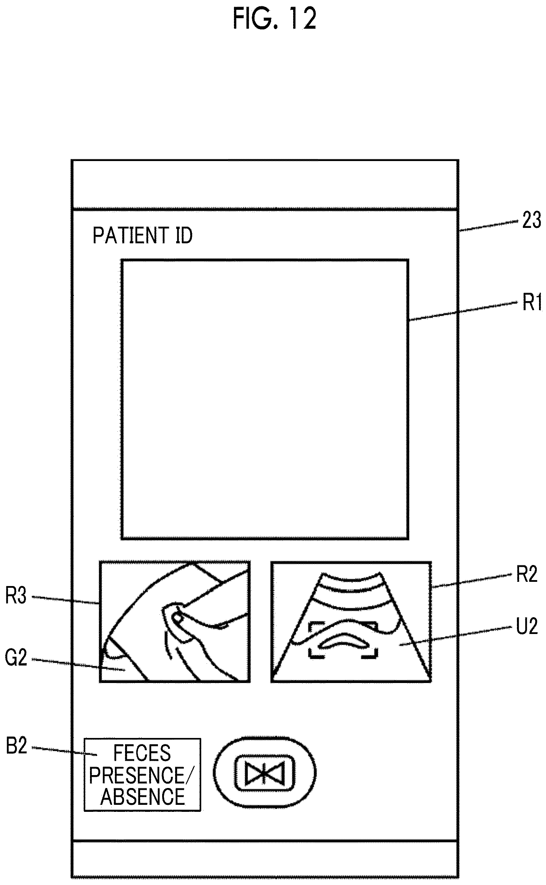

In a case where the past ultrasound image U2 of the subject specified by the subject specification unit 30 is stored in the image memory 24 and the optical image stored in correspondence with the past ultrasound image U2 is present, the scanning guide display unit 27 displays the optical image corresponding to the past ultrasound image U2 in the third display region R3 of the monitor 23 as a scanning guide G2, as shown in Fig. 12.

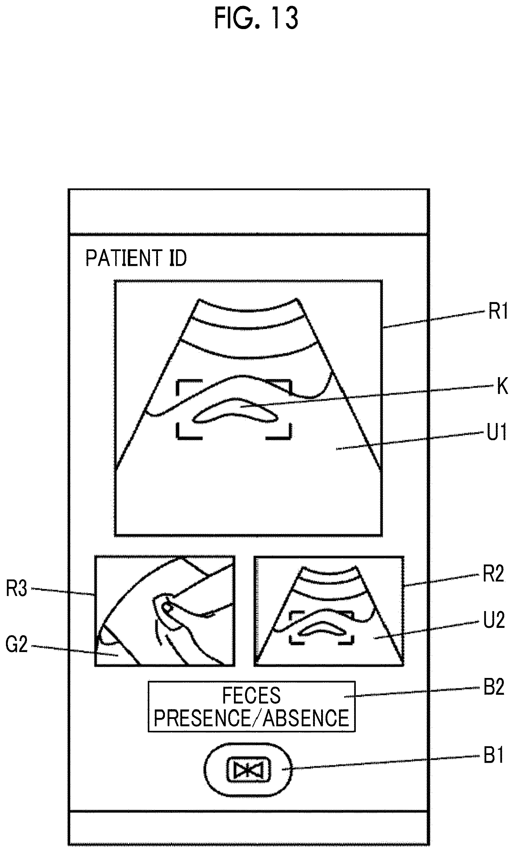

In addition, in a case where the processing of detecting the feces K is executed by the feces detection unit 28, the ultrasound image U1 in which the feces K is highlighted and displayed is displayed in the first display region R1 together with the past ultrasound image U2 displayed in the second display region R2 and the optical image displayed in the third display region R3, as shown in Fig. 13.

From the above, with the ultrasound diagnostic system of Embodiment 2, the scanning guide display unit 27 displays the past optical image corresponding to the past ultrasound image U2 in the third display region R3 as the scanning guide G2. Therefore, the user can easily image the observation site in the same technique as the technique in the past examination in which the observation site can be clearly imaged, by confirming the past optical image displayed as the scanning guide G2 in the third display region R3.

### Explanation of References

1: ultrasound probe
2, 2A: apparatus main body
11: transducer array
12: transmission and reception circuit
21: image generation unit
22: display controller
23: monitor
24: image memory
25: observation site selection unit
26: reference image display unit
27: scanning guide display unit
28: feces detection unit
29: past image display unit
30: subject specification unit
31, 31A: main body controller
32: input device
33: image acquisition unit
34, 34A: processor
41: pulsar
42: amplification section
43: AD conversion section
44: beam former
45: signal processing section
46: DSC
47: image processing section
61: optical camera
A1 to A8: icon
B1: freeze button
B2: feces presence/absence button
B3: not-apply button
B4: rescan button
C1 to C3: annotation icon
G1, G2: scanning guide
K: feces
L1: region-of-interest suggestion line
M1: menu screen
R1: first display region
R2: second display region
R3: third display region
U1, U2: ultrasound image
UR: reference image

## Claims

1. An ultrasound diagnostic system that displays an ultrasound image of an observation site of a subject captured by scanning with an ultrasound probe (1), the ultrasound diagnostic system comprising:
a subject specification unit (30) configured to specify the subject in response to an input of identification information of the subject;
an observation site selection unit (25) configured to select the observation site;
a monitor (23) having a first display region and a second display region;
a display controller (22) configured to display the captured ultrasound image in the first display region;
a reference image display unit (26) configured to display, in the second display region, a reference image for the observation site selected by the observation site selection unit (25);
an image memory (24) configured to store a past ultrasound image of the subject; and
a past image display unit (29) configured to, in a case where it is determined whether or not the past ultrasound image of the observation site of the subject specified by the subject specification unit (30) is stored in the image memory (24), and the past ultrasound image is stored, read out the past ultrasound image from the image memory (24) and display the past ultrasound image in the second display region instead of the reference image.

2. The ultrasound diagnostic system according to claim 1,
wherein the past image display unit (29) is configured to, in a case where a plurality of the past ultrasound images are stored in the image memory (24), select a past ultrasound image closest to the ultrasound image being displayed in the first display region from the plurality of past ultrasound images and display the selected past ultrasound image in the second display region.

3. The ultrasound diagnostic system according to claim 1 or 2,
wherein the monitor (23) has a third display region different from the first display region and the second display region, and
the ultrasound diagnostic system further comprises a scanning guide display unit (27) configured to display, in the third display region, a scanning guide for guiding scanning of the ultrasound probe (1) for the observation site selected by the observation site selection unit (25).

4. The ultrasound diagnostic system according to claim 3,
wherein the scanning guide is a video.

5. The ultrasound diagnostic system according to claim 3,
wherein the scanning guide is an optical image in which the subject and the ultrasound probe (1) during the scanning of the observation site of the subject are captured.

6. The ultrasound diagnostic system according to claim 5, further comprising:
an optical camera (61) configured to acquire the optical image.

7. The ultrasound diagnostic system according to any one of claims 1 to 6,
wherein the observation site is any one of an entire large intestine, a colon, or a rectum.

8. The ultrasound diagnostic system according to claim 7, further comprising:
a feces detection unit (28) configured to detect a feces from the ultrasound image,
wherein the display controller (22) is configured to highlight and display, in the ultrasound image, a position of the feces detected by the feces detection unit (28).

9. The ultrasound diagnostic system according to claim 8,
wherein the feces detection unit (28) is configured to detect the feces by using a trained model that has been trained using ultrasound images of large intestines of a large number of subjects.

10. A control method for an ultrasound diagnostic system, the control method being for displaying an ultrasound image of an observation site of a subject captured by scanning with an ultrasound probe (1), comprising:
specifying the subject in response to an input of identification information of the subj ect;
selecting the observation site;
displaying the captured ultrasound image in a first display region of a monitor (23);
displaying a reference image for the selected observation site in a second display region of the monitor (23);
storing a past ultrasound image of the subject in an image memory (24); and
in a case where it is determined whether or not the past ultrasound image of the observation site of the specified subject is stored in the image memory (24), and the past ultrasound image is stored, reading out the past ultrasound image from the image memory (24) and displaying the past ultrasound image in the second display region instead of the reference image.
